(19) Europäisches Patentamt
European Patent Office
Office européen des brevets

(11) Publication number: **0 095 362**
**A1**

(12) # EUROPEAN PATENT APPLICATION

(21) Application number: 83302936.6

(22) Date of filing: 23.05.83

(51) Int. Cl.$^3$: C 12 P 19/44
C 07 H 15/26, C 07 H 7/02

(30) Priority: 24.05.82 US 381528
14.07.82 US 398212
20.08.82 US 409986

(43) Date of publication of application:
30.11.83 Bulletin 83/48

(84) Designated Contracting States:
AT BE CH DE FR GB IT LI LU NL SE

(71) Applicant: REPLIGEN CORPORATION
101 Binney Street
Cambridge Massachusetts 02142(US)

(72) Inventor: Fraser, Thomas H.
44 Bullough Park
Newtonville Massachusetts 02160(US)

(72) Inventor: Herlihy, Walter C.
648 Huron Avenue
Cambridge Massachusetts 02138(US)

(74) Representative: Holdcroft, James Gerald, Dr. et al,
Graham Watt & Co. Riverhead
Sevenoaks Kent TN13 2BN(GB)

(54) **Glucuronides and methods for their preparation.**

(57) *In vitro* enzymatic processes are disclosed which efficiently convert compounds containing a primary alcohol to their corresponding novel O-glucuronic acid derivatives. Exemplified as starting compounds are anticholinergics containing a primary alcohol. The novel glucuronides are useful as UV absorbents.

EP 0 095 362 A1

# GLUCURONIDES AND METHODS FOR THEIR PREPARATION

The preparation of β-glucuronides has been carried out by a number of different techniques. Chemical synthesis typically involves condensation of a suitably protected aglycon with an alkyl (2,3,4-tri-O-acetyl-β-D-glucopyranosyl halide) glucuronate followed by deprotection of the glucuronide and aglycon (Ando, K., Suzuki, S., and Arita, M. [1970] J. Antibiotics 23, 408; Sarett, L.H., Strachan, R.G., and Hirschmass, R.F. [1966] U.S. Patent 3,240,777). A second approach involves feeding large amounts of the aglycon to animals, collecting their urine and isolating the glucuronide (Hornke, I., Fehlhaber, H.W., Uihlein, M. [1979] U.S. Patent 4,153,697). Alternatively, the animal can be sacrificed and the bile isolated from its gall bladder from which the glucuronide is purified (DeLuca, H.F., Schnoes, H.K., and LeVan, L.W. [1981] U.S. Patent 4,292,250). This *in vivo* synthesis is catalyzed by the class of enzymes known as uridine diphosoglucuronyl transferases. *In vitro* use of the enzyme to produce various β-glucuronides had been reported; for example, a phenolic compound has been glucuronidated (Johnson, D.B., Swanson, M.J., Barker, C.W., Fanska, C.B., and Murrill, E.E. [1979] Prep. Biochem. 9, 391).

An *in vitro* enzymatic process for the synthesis of β-glucuronides has several advantages over prior art chemical synthesis or animal feeding methods. Chemical synthesis requires a minimum of four steps: (1) protection of all the nucleophilic groups in the aglycon except the one involved in the glycosidic linkage, (2) preparation of a suitably protected reactive derivative of D-glucuronic acid, e.g., methyl (2,3,4-tri-O-acetyl-β-D-glucopyranosyl halide) glucuronate, (3) condensation, and (4) deprotection. Complications arise if the aglycon contains functional groups sensitive to the conditions of deprotection. For example, aglycons containing esters or other alkali-sensitive linkages can be hydrolyzed during the saponification of the methyl and acetyl protecting groups.

The animal feeding approach to making β-glucuronides also has several disadvantages as compared to an *in vitro* enzymatic method. The most significant disadvantage is that stringent purification is required.

In contrast, an *in vitro* enzymatic process involves a *single step* condensation between a readily available cofactor and the aglycon.

The present invention provides a process for preparing the O-β-D-glucuronic acid of a compound having a primary alcohol moiety characterised by converting said compound having a primary alcohol moiety to its corresponding O-glucuronic acid derivative by an *in vitro* enzymatic process.

The present invention also provides a process for separating ammonium (+)-tropicamide O-β-D-glucuronic acid and ammonium (-)-tropicamide O-β-D-glucuronic acid from ammonium (+),(-)-tropicamide O-β-D-glucuronic acid characterised by subjecting

ammonium (+),(-)-tropicamide O-$\beta$-D-glucuronic acid to reversed phase liquid chromatography.

We have found, for example, that upon incubating liver microsomes in the presence of a suitable buffer to maintain the pH at about 7 to about 8.5, (+,-)-tropicamide, and uridine 5'-diphosphoglucuronic acid (UDPGA), for a

sufficient time, there is obtained a preparation of (+),(-)-tropicamide O-β-D-glucuronide. An ammonium salt mixture of these glucuronides can be isolated in its essentially pure form by reversed phase chromatography. The diastereomers can be completely resolved to their essentially pure forms by a high pressure liquid chromatographic (HPLC) system disclosed herein.

Novel glucuronides of ester-containing anticholinergics are prepared by first removing all of substantially all of the esterase activity from liver microsomes. These esterases are removed since they will hydrolyze the aglycon and/or its glucuronic acid derivative. This operation can be done by washing the liver microsomes in a suitable buffer, as described herein, or by other equivalent washing means known to persons in this art. Advantageously, an esterase inhibitor can be used to supplement the washing of the microsomes. For example, a competitive inhibitor of the esterases such as lysine ethyl ester, and the like, or a suicide substrate such as phenylmethylsulfonyl fluoride, and the like, can be used. The thus obtained liver microsomes are then incubated for a sufficient length of time with the following:

(1) a suitable buffer to maintain the pH at about 7 to about 8.5;

(2) an ester-containing anticholinergic having a primary alcohol; and

(3) UDPGA (uridine 5'-diphosphoglucuronic acid).

It will be realised that the esterase inhibitor is retained in the microsomes so that it prevents any residual esterase activity in the incubation step.

A sufficient length of time for incubation is that which allows the conjugation of the aglycon with glucuronic acid.

The above processes utilize the cofactor uridine 5'-diphosphoglucuronic acid (UDPGA). The following process, advantageously, eliminates the need for this relatively expensive cofactor.

Upon reacting a solution of D-glucuronic acid with a solution of a compound which has a primary alcohol, and a solution of β-glucuronidase for a time sufficient to form the glucuronide, there is obtained the O-β-D-glucuronide of said compound. More specifically, a solution of glucuronic acid is reacted with a solution of a compound, e.g., tropicamide, scopolamine, hyoscyamine, atropine, and like acceptor substrates which have a primary alcohol, and a solution of β-glucuronidase, to give the O-β-D-glucuronide of said compound. Any β-glucuronidase can be used in the process, e.g., E. coli, bovine liver, Mollusk, and the like. These named are perhaps the most readily available β-glucuronidases. A wide range of concentration of reactants can be used in the process so long as the particular reactant is in solution. Advantageously, the higher the concentration of glucuronic acid, the higher the yield of the desired O-β-D-glucuronide.

The invention also comprehends the products of a process according to the invention; and base addition salts thereof.

Compounds embodying the invention are useful because of their absorption of ultraviolet light.

They can be used as ultraviolet absorbents in technical and industrial areas as follows:

(a) Textile materials; such textile materials may consist of natural materials of animal origin, such as wool or silk, or of vegetable origin, such as cellulosic materials of cotton, hemp, flax, or linen, and also semi-synthetic materials, such as regenerated cellulose, for example, artificial silk viscoses, including staple fibers of regenerated cellulose.

(b) Fibrous materials of other kinds (that is to say, not textile materials) which may be of animal original, such as feathers, hair, straw, wood, wood pulp or fibrous materials consisting of compacted fibers, such as paper, cardboard or compressed wood, and also materials made from the latter; and also paper masses, for example, hollander masses, used for making paper.

(c) Coating or dressing agent for textiles or paper.

(d) Lacquers or films of various compositions.

(e) Natural or synthetic resins.

(f) Natural rubber-like materials.

(g) Filter layers for photographic purposes, especially for color photography.

Depending on the nature of the material to be treated, the requirements with regard to the degree of activity and durability, and other factors, the proportion of the light-screening agent to be incorporated in the material may vary

within fairly wide limits, for example, from about 0.01% to 10%, and, advantageously, 0.1% to 2%, of the weight of the material which is to be directly protected against the action of ultraviolet rays.

The glucuronides demostrate enhanced water solubility. This property can be advantageous for the pharmaceutical use of medicinals, for example, mycophenolic acid glucuronide. See U.S. Patents 3,777,020 and 3,758,455.

Other uses for glucuronides are as cardiotonic agents (U.S. 4,335,131); vitamin D derivatives (U.S. 4,292,250); gastric acid secretion inhibitors (European Patent Application 52-074); and as antitumor agents (U.S. 3,758,455).

The above enzymatic process for the glucuronidation of (+,-)-tropicamide involving UDPGA was unexpectedly successful in view of the fact that attempts to glucuronidate another primary alcohol, i.e. (-)-scopolamine, by this method were unsuccessful. Also, there is no known prior art which discloses the preparation of essentially pure(+),(-)-tropicamide O-$\beta$-D-glucuronide and (-)-tropicamide O-$\beta$-D-glucuronide. The subject process is particularly advantageous because the reaction yields a single pair of stereospecific products, as disclosed above.

The above enzymatic process for the glucuronidation of ester-containing anticholinergics involving UDPGA was unexpectedly successful in view of the fact that prior attempts to glucuronidate the ester-containing anticholinergic scopolamine were unsuccessful. We believe this process is the first known <u>in vitro</u> enzymatic process for preparing glucuronides of ester-containing anticholinergics having a primary alcohol.

The enzymatic reaction, described herein, can be carried out over a pH range of about 7 to about 8.5

with different buffer strengths and with various buffers, for example, sodium N-2-hydroxyethyl piperazine-N'-2-ethanesulfonic acid, 3-((tris-(hydroxymethyl) methyl)amino)propane sulfonic acid, tris hydrochloride, and the like. Quantitative glucuronidation of (+,-)-tropicamide can be obtained by increasing the amount of UDP glucuronic acid in the reaction.

Liver microsomes which can be used can be obtained from animal sources, for example, rabbit, bovine, rat, and the like.

The temperature of incubation in the enzymatic step can be from about 20° to about 45°C.

As disclosed above, as well as the processes using the cofactor uridine 5'-diphosphoglucuronic acid (UDPGA) we have developed a process which eliminates the need for this cofactor. It not only dispenses with the use of UDPGA by the use of a very inexpensive reactant, but, advantageously, it can be used to prepare the O-β-D-glucuronide of any compound containing a primary alcohol.

Unexpectedly, the O-β-D-glucuronide of phenols cleavable by β-glucuronidase, as well as secondary and tertiary alcohols, cannot be prepared in this way. We believe this process is the only known process which utilizes reversal of hydrolysis by β-glucuronidase in an <u>in vitro</u> biosynthetic reaction.

The chromatographic methods used are based on reversed phase liquid chromatography on e.g. C-18 silica supports. This technique is well suited for the purification of enzymatically-produced glucuronides of hydrophobic compounds. Unreacted aglycon is much more hydrophobic than the corresponding glucuronide and thus will be well resolved on reversed phase systems. The cofactor, UDP glucuronic acid, and the byproduct, UDP, are both very hydrophilic and will be much less retained than the glucuronide of a hydrophobic compound. Finally, all the solvent systems described are based on $NH_4OAc$, a volatile buffer. Modifications to this system may be necessary in order to purify glucuronides of very hydrophilic compounds. Other reversed phase stationary supports, for example phenyl silica, C-8 silica, and the like, can be used. The resolution of the two tropicamide diastereomers is enhanced when the pH is lowered from 7.0 to 3.7, which would increase the fraction of the molecules in the zwitterionic form necessary for an intramolecular ionic interaction. In addition, increasing the ionic strength from 0.1% $NH_4OAc$ to 1% $NH_4OAc$

diminishes the resolution as would be expected if an intramolecular "salt bridge" were present.

The following Examples are illustrative of the process and products of the invention, but are not to be construed as limiting. All percentages are by weight and all solvent mixture proportions are by volume unless otherwise noted.

Example 1--Enzymatic preparation of (+,-)-tropicamide O-β-D-glucuronide.

Four grams of a rabbit liver or bovine liver microsomal fraction (Sigma Chemical Co., St. Louis, Mo.) are suspended in 100 ml of a 75 mM tris hydrochloride buffer (pH=7.5-8.0). The microsomes are suspended by repeatedly drawing the mixture through a pipette tip. The microsomes are then pelleted by centrifugation at 100,000 g for 30 minutes. The supernatant is discarded, and the pellet is resuspended to 100 ml with a 150 mM tris hydrochloride (pH=7.5-8.0) solution, containing 200 mg (+,-)-tropicamide (Hoffman-LaRoche, Nutley, N.J., also disclosed in U.S. Patent 2,726,245) and 1 gram of sodium uridine 5'-diphosphoglucuronic acid (Sigma Chemical Co.). After a 20 hr. incubation at 37°C, the reaction is terminated by heating to about 70°C, and centrifuging the reaction mixture. The desired product is in the supernatant. The yield of desired product is determined by high pressure liquid chromotography (HPLC) to be ~75%.

The HPLC conditions are as follows: a .39 x 30 cm C-18 μBondapak column (Waters Associates,

Milford, Mass.) is eluted at 2 ml/min. with .1% $NH_4OAc$ (pH=5.75). After injection of the sample, a linear gradient to 60% methanol is applied to the column over a 20-minute period. The column eluant is monitored with an ultraviolet detector set at 254 nm. Under these conditions the reaction product elutes as a partially resolved doublet. On the basis of the chemical and spectral data presented below the two peaks are assigned as (+)-tropicamide O-β-D-glucuronide and (-)-tropicamide O-β-D-glucuronide.

Example 2--Isolation of essentially pure (+), (-)-tropicamide 0-β-D-glucuronide.

The pH of the reaction mixture, obtained in Example 1, is adjusted to 5.75 with 1.26 ml of 10% $NH_4OAc$ (pH=5.75); 25 ml of methanol is added to the reaction, and the suspension is centrifuged at 44,000 g for 60 minutes. The supernatant is collected and loaded onto a 15 mm by 250 cm column of octadecyl derivatized silica (50-100 μ particles) (Waters Associates) which had been equilibrated with an 80/20 solution of .1% $NH_4OAc$ (pH=5.75)/methanol. The column is washed at 3 ml/min. until the absorbance of the eluant at 254 nm is les than .05. Essentially pure (+),(-)-tropicamide O-β-D-glucuronide is then eluted with a 55/45 solution of .1% $NH_4OAc$ (pH=5.75)/methanol. Unreacted (+,-)-tropicamide is eluted from the column with a 40/60 solution of .1% $NH_4OAc$ (pH=5.75)/methanol. The desired product contains less than 1% of (+,-)-tropicamide contamination.

Example 3--Separation of (+)- and (-)-tropicamide O-β-D-glucuronide.

The two isomers are isolated from the mixture obtained in Example 2 as follows:

The two isomers by HPLC on a .39 x 30 cm column of C-18 μBondapak (Waters Associates). The column is equilibrated with .013 M $NH_4OAc$ (pH=3.7) containing 10% methanol at a flow rate of 2 ml/min. One minute after injection of the sample, the percentage of methanol in the eluant is raised to 22% in one minute. The two diastereomers elute at about eleven and theirteen minutes, respectively. Retention times vary with column condition and the optimal concentration of methanol is normally determined with analytical injections. The two diastereomers are obtained in their essentially pure form.

Characterization of (+)- and (-)-tropicamide O-β-D-glucuronide.

The two reactionproducts (50 ug in 150 ul of 50 mM sodium phosphate, pH=6.8) are individually treated with ten Fishman units of E. coli β-glucuronidase (EC 3.2.1.31) at 37°C for 1 hour. Both compounds are quantitatively hydrolyzed by the glucuronidase to products which were indistinguishable by HPLC from the starting material, (+,-)-tropicamide, in the .1% $NH_4OAc$ (pH=5.75)/methanol solvent system described above. The products are also indistinguishable from (+,-)-tropicamide when chromatographed on C-18 in a second solvent system consisting of 1%

triethylammonium acetate (pH=7.0) eluted with a linear gradient to 50% acetonitrile in 25 minutes. These data show that both products contain an intact tropicamide moiety. The known specificity of the enzyme shows the presence of a glucuronic acid moiety and shows that the glycosidic linkage has the β configuration. The tropicamides released by glucuronidase treatment are individually converted back to the corresponding glucuronides using the conditions described above. These reactions produced single products, i.e., the tropicamide derived from glucuronidase treatment of component 1 yields only component 1, and the tropicamide derived from component 2 yields only component 2. Thus the two products are diastereomers which differ only in the configuration of the optically active carbon in the tropicamide moiety.

The products of β-glucuronidase hydrolysis are further characterized by their rotation of 589 nm plane polarized light. These measurements show that the component which elutes earlier in the HPLC assay is dextrorotatory and the later eluting compound is levorotatory. Experiments with lesser amounts of E. coli glucuronidase show that the hydrolysis rate of (+)-tropicamide O-β-D-glucuronide is approximately twice as rapid as (-)-tropicamide O-β-D-glucuronide.

The ultraviolet spectra of (+),(-)-tropicamide, (+)-tropicamide O-β-D-glucuronide, and (-)-tropicamide O-β-D-glucuronide are recorded in

a .05% $NH_4OAc$ pH=7.0) solution. All three samples have identical spectra with maxima at 257 nm (Emax=2140) and shoulders at 252 nm and 263 nm characteristic of a para substituted pyridone moiety.

The molecular weights of the two diastereomers are determined by direct chemical ionization (DCI) mass spectrometry and fast atom bombardment (FAB) mass spectrometry. The ammonia DCI spectrum of each isomer gives a quasi molecular ion at m/z=461 (M+H)+, confirming the molecular weight as 460. Similarly the zenon FAB sepctrum of both isomers contains a series of ions at m/z=461 (M+H)+, m/z=483 (M+Na)+, and m/z=499 (M+K)+ clearly showing a molecular weight of 460.

The infrared spectra in KBr pellets of the two tropicamide glucuronides both exhibit strong absorption bands centered at 3150 $cm^{-1}$ and 1400 $cm^{-1}$ confirming that the ammonium salt had been formed as expected. Both compounds also exhibit a broad band at 1600 $cm^{-1}$ which is consistent with the presence of both a carboxylate and a tertiary amide carbonyl. In addition, a shoulder at 3550 $cm^{-1}$ is consistent with the hydroxyl groups in the glucuronides.

The ammonium and other base salts of the compounds are useful in the same manner as the free acid form. If desired the ammonium salt can be converted to the free acid by means well known in the art, for example, by adjusting the pH of the ammonium salt solution with a weak acid so as

not to cause hydrolysis of the diastereomer. Salts with both inorganic and organic bases can be formed with the free acid. For example, in addition to ammonium salt, there also can be formed the sodium, potassium, calcium, and the like, by neutralizing an aqueous solution of the free acid.

(+)(-)-Tropicamide O-β-D-glucuronic acid has the following formula:

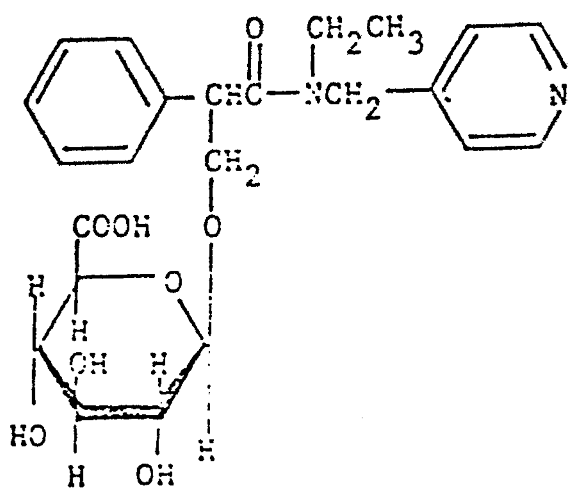

Example 4--Preparation of scopolamine O-β-D-glucuronic acid.

Four hundred milligrams of rabbit liver or bovine liver microsomal fraction (Sigma Chemical Co.), containing uridine 5'-diphosphoglucuronyl transferase, is suspended in 20 ml of a 75 mM tris HCl buffer (pH=8.0). The microsomes are suspended by repeatedly drawing the mixture through a pipette tip. The microsomes are then pelleted by centrifugation at 44,000 g for 20 minutes. The supernatant is discarded, the pellet washed a second time, and the pellet resuspended to 10 ml

with a 75 mM tris HCl (pH=8.0) solution containing 20 mg scopolamine (Sigma) and 140 mg of sodium uridine 5'-diphosphoglucuronic acid (Sigma). In addition, the reaction mixture contains either 100 mM lysine ethyl ester (Sigma) or 10 μM phenylmethylsulfonyl fluoride (PMSF) (Sigma) which had been predissolved in a small volume of propanol immediately before addition. After a 20-hour incubation at 37°C, the reaction is terminated by heating the sample for two minutes at 70°C, followed by centrifugation at 44,000 g for 20 minutes. The supernatant is removed and analyzed by HPLC. The yield of desired product is determined to be ~95%.

The HPLC conditions are as follows: a .39 x 30 cm C-18 μBondapak column (Waters Associates) is eluted at 2 ml/min. with .1% $NH_4OAc$ (pH=7.5). After injection of the sample, a linear gradient to 60% methanol is applied to the column over a 20-minute period. The column eluant is monitored with an ultraviolet detector set at 254 nm. Under these conditions the reaction product has a retention time of ~12 minutes, whereas scopolamine has a retention time of ~18 minutes. On the basis of the chemical and spectral data presented below, the product is assigned as scopolamine O-β-D-glucuronide.

<u>Example 5--Preparation of hyoscyamine O-β-D-glucuronic acid.</u>

The reaction conditions are identical to those utilized for scopolamine in Example 4. The concentration of hyoscyamine is 2 mg/ml and the reaction is carried out for 20 hours.

Example 6--Isolation of scopolamine O-β-D-glucuronide and hyoscyamine O-β-D-glucuronic acid.

The glucuronides are isolated with the HPLC system described above. Typically, 25 µl of 1% $NH_4OAc$ (pH=7.5) is added to 225 µl of the reaction supernatant, and the entire sample is injected. Larger amounts can be prepared with a preparative chromatography system.

Characterization of scopolamine O-β-D-glucuronic acid.

The reaction product (150 µg in 450 µl of 50 mM sodium phosphate, pH=6.8) is treated with 150 Fishman units of *E. coli* β-glucuronidase (EC 3.2.1.31) at 37°C for two hours. The compound is quantitatively hydrolyzed by the glucuronidase to product which is indistinguishable by HPLC from the starting material, scopolamine, in the .1% $NH_4OAc$ (pH=7.5)/methanol solvent system described above. The glucuronidase product is also indistinguishable from scopolamine when chromatographed on C-18 in a second solvent system consisting of 1% triethylammonium acetate (pH=7.0) eluted with a linear gradient to 50% acetonitrile in 25 minutes. Since the chromatographic behavior of scopolamine is markedly affected by pH in the range of pH=5-8, the hydrolysis product is chromatographed in a third solvent system consisting of .1% $NH_4OAc$ (pH=5.0) eluted with a linear gradient to 50% acetonitrile in 25 minutes and found to be identical to scopolamine. These data indicate that the product contains an intact scopolamine moiety.

The known specificity of this enzyme indicates the presence of a glucuronic acid moiety and indicates that the glycosidic linkage has the β configuration.

The ultraviolet spectrum of the reaction product is recorded in .05% $NH_4OAc$ (pH=7.0) and compared to the spectrum of scopolamine. Both compounds exhibit maxima at 252 nm, 258 nm, and 263.5~nm, and a strong end absorption beginning at 240 nm, indicating that the glucuronide contains an intact tropic acid moiety.

The molecular weight of the product is determined by fast atom bombardment (FAB) mass spectrometry. The xenon FAB spectrum contained a single ion at m/z=480 (M+H)+, clearly indicating a molecular weight of 479. The exact mass of the (M+H)+ ion is determined by peak matching to be 480.186, which is in excellent agreement with the mass expected for a compound with this elemental composition, 480.187.

Scopolamine O-β-D-glucuronic acid has the following structure:

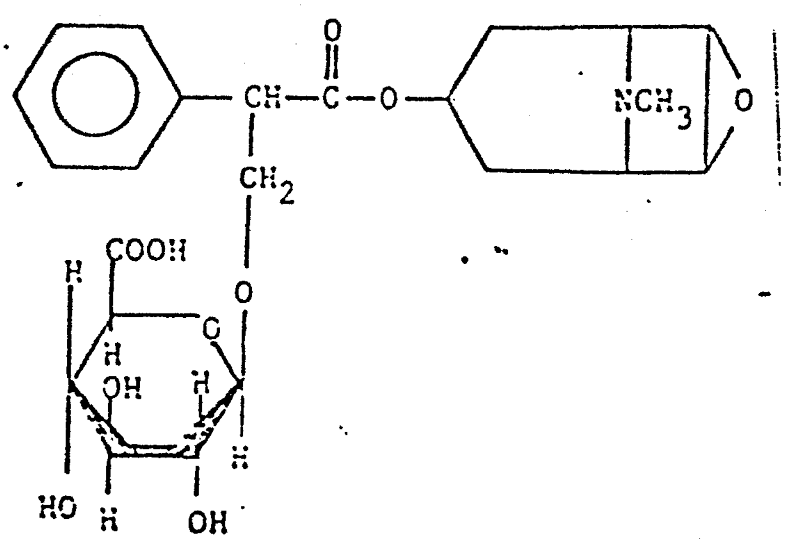

Characterization of hyoscyamine O-β-D-glucuronic acid.

The HPLC system used to assay the synthesis of hyoscyamine O-β-D-glucuronic acid consists of a linear gradient from .1% $NH_4OAc$ (pH=5.75) to 60% methanol in twenty minutes. All other parameters are identical to the chromatography described above for scopolamine. Under these conditions the hyoscyamine elutes slightly after scopolamine, and the product of the transferase reaction elutes slightly after scopolamine O-β-D-glucuronic acid, indicating that the expected glucuronide is formed. This product is purified by HPLC. Approximately 40 µg is dissolved in 400 µl of 50 mM sodium phosphate (pH=6.8) containing 1000 U/ml of *E. coli* β-glucuronidase. Immediately after addition of the enzyme and after a one-hour incubation at 37°C, 50 µl aliquots are removed, heated at 70°C for 1 minute and analyzed by HPLC. The aglycon released from the glucuronide and hyoscyamine have identical retention in the .1% $NH_4OAc$ (pH=5.75)/methanol solvent system described above.

Hyoscyamine O-β-D-glucuronic acid has the following structure:

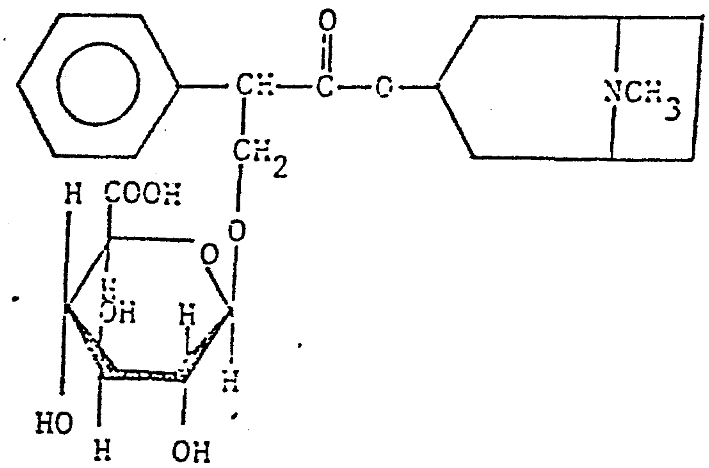

Esterase cleavage of scopolamine and scopolamine O-β-D-glucuronic acid.

To 150 µl of 150 mM tris HCl (pH=8.0) solution containing .7 mM scopolamine and an equimolar amount of scopolamine O-β-D-glucuronic acid is added 1 mg of unwashed rabbit UDPGA-dependent glucuronyl transferase. Immediately after addition of the enyzme, 50 µl are removed and incubated at 70°C for 1 minute and centrifuged at 14,000 g for 5 minutes. The supernatant (40 µl) is removed; 4 µl of 1% $NH_4OAc$ (pH=7.5) is added, and the sample is analyzed by HPLC using the .1% $NH_4OAc$ (pH=7.5)/methanol solvent system described above. A second 50 µl sample is prepared and analyzed after a 2-hour incubation at 37°C.

Example 7.

Upon substituting atropine in Example 5 for hyoscyamine, there is obtained atropine O-β-D-glucuronic acid.

Example 8.

Upon substituting other ester-containing anticholinergics having a primary alcohol in Example 4 for scopolamine, there are obtained the corresponding ester-containing anticholinergic O-β-D-glucuronic acids.

Example 9.

Salts with both inorganic and organic bases can be formed with the free acid of the compounds embodying the invention. For example, in addition to the ammonium salt, there also can be formed the sodium, potassium, calcium, and the like, by neutralizing an aqueous solution of the free acid with the corresponding base. The ammonium and other base salts of the compounds embodying the invention are useful in the same manner as the free acid form.

Example 10--Synthesis of (+,-) tropicamide O-β-D-glucuronic acid by reverse hydrolysis.

A 1.7 M sodium D-glucuronic acid stock solution is prepared by adding 16.5 gm of glucuronic acid to 40 ml of 50 mM sodium phosphate buffer; the pH is adjusted to ~6.8 with 5 N NaOH, and the final volume is adjusted to 50 ml with sodium 50 mM sodium phosphate (pH=6.8).

A 300 µl enzyme reaction is prepared by combining 100 µl of the 1.7 M solution of sodium D-glucuronic acid, 100 µl of a 5 mg/ml solution of

tropicamide, 30 µl of a .5 M solution of sodium phosphate (pH=6.8), 50 µl of water and 20 µl of a 1000 Unit/ml solution of freshly dissolved E. coli β-glucuronidase (E.C. 3.2.1.31) (Sigma Type III, Sigma Chemical Co., St. Louis, Mo.). Immediately after addition of the enzyme, a 25 µl aliquot is removed and incubated at 37°C. Both samples are diluted with an equal volume of .1% $NH_4OAc$ (pH=-5.75) and analyzed by high pressure liquid chromatography (HPLC) as follows: a .39 x 30 cm C-18 µBondapak column (Waters Associates, Milford, Mass.) is eluted at 2 ml/min. with .1% $NH_4OAc$ (pH=5.75). After injection of the sample, a linear gradient to 60% methanal is applied to the column over a 20 minute period. The column eluant is monitored with an ultraviolet detector set at 254 nm. Approximately 1% of the (+,-)-tropicamide is converted to the corresponding O-β-D-glucuronic acid derivative.

Example 11--Separation of (+)- and (-)-tropicamide O-β-D-glucuronide.

The two isomers are isolated from a mixture and characterized as disclosed in Example 3.

The ammonium and other base salts of the compounds are useful in the same manner as the free acid form. If desired the ammonium salt can be converted to the free acid as described in Example 3.

Example 12--Synthesis of scopolamine O- -D-glucuronic acid by reverse hydrolysis.

To a vial containing 1000 Units of lyophilized E. coli β-glucuronidase is added 300 µl of a 100 mg/ml solution of scopolamine, 2.4 ml of the 1.7 M sodium glucuronic acid stock solution described in Example 10, and 300 µl of a 0.5 M sodium phosphate solution (pH=6.8). Samples (50 µl) are taken immediately after mixing and after a 20-hour incubation at 37°C. The enzyme is inactivated by heating as described in Example 10. Samples are diluted with an equal volume of 0.1% $NH_4OAc$ (pH=7.5) and analyzed by HPLC using the system described in Example 4. This product is characterized as described in Example 6.

Example 13--Synthesis of tropicamide O-β-D-glucuronic acid with bovine liver β-glucuronidase by reverse hydrolysis.

a 1.2 ml solution containing 0.85 M glucuronic acid, 4 mg/ml tropicamide, 48,00 Units of bovine liver β-glucuronidase (Sigma Type B-1, Sigma Chemical Co.) and 50 mM NaOAc (pH=5.0) is incubated at 37°C. After 20 hours 50 µl is removed and heated at 70°C for one minute, spiked with 5 µl of 1% $NH_4OAc$ (pH=7.5) and analyzed by HPLC using the conditions described above for the analysis of scopolamine glucuronic acid. The product of the reaction has an identical retention time as standard (+,-)-tropicamide O-β-D-glucuronic acid. Approximately 5 µg of product is isolated by HPLC of which 2 µg is dissolved in 122 ul of 50 mM sodium phosphate (pH=6.8), to which is added 122 ul of a 1000 Unit/ml solution of E. coli

β-glucuronidase. The product is quantitatively converted to tropicamide after a 10-minute incubation at 37°C judged by HPLC analysis.

Example 14--Synthesis of tropicamide O-β-D-glucuronic acid with Mollusk β-glucuronidase by reverse hydrolysis.

A 1 ml solution containing 4 mg tropicamide and 1.2 M glucuronic acid is adjusted to pH=3.8 with concentrated hydrochloric acid. This solution is combined with a 1 ml solution containing 4064 Units of Abalone β-glucuronidase (Sigma) in 50 mM sodium acetate (pH=3.8). After a 16-hour incubation, a 50 μl aliquot is heated and analyzed by HPLC as described above for the bovine β-glucuronidase reaction. A product peak with retention time equal to that of tropicamide O-β-D-glucuronic acid standard is observed.

Example 15.

Upon substituting atropine in Example 12 for scopolamine, there is obtained atropine O-β-D-glucuronic acid.

Example 16.

Upon substituting hyoscyamine in Example 12 for scopolamine, there is obtained hyoscyamine O-β-D-glucuronic acid.

Example 17.

Upon substituting other acceptor substrates which have a primary alcohol for tropicamide in Example 10, or in Example 12 for scopolamine, there is obtained the corresponding glucuronide of the acceptor substrate used in the reaction.

Example 18.

Upon substituting any anticholinergic having a primary alcohol for tropicamide in Example 10, or in Example 12 for scopolamine, there is obtained the corresponding glucuronide of said anticholinergic.

Example 19.

Salts with both inorganic and organic bases can be formed with the free acid of the compounds prepared by the subject process. For example, in addition to the ammonium salt, there also can be formed the sodium, potassium, calcium, and the like, by neutralizing an aqueous solution of free acid with the corresponding base. The ammonium and other base salts of the compounds are useful in the same manner as the free acid forms.

CLAIMS:

1. A process for preparing the O-β-D-glucuronic acid of a compound having a primary alcohol moiety characterised by converting said compound having a primary alcohol moiety to its corresponding O-glucuronic acid derivative by an *in vitro* enzymatic process.

2. A process according to claim 1, wherein the compound having a primary alcohol moiety is an anticholinergic compound.

3. A process according to claim 2, wherein the cholinergic compound is selected from tropicamide, scopolamine, atropine, and hyoscyamine.

4. A process according to claim 2, which comprises:

(a) incubating liver microsomes at a suitable temperature in the presence of a suitable buffer at a suitable pH, with
(i) the anticholinergic compound, and
(ii) uridine 5'-diphosphoglucuronic acid, for a sufficient time to conjugate the aglycon with glucuronic acid, and
(b) isolating the O-β-D-glucuronic acid of the anticholinergic compound from the reaction mixture.

5. A process, according to claim 4, wherein said O-β-D-glucuronic acid is essentially pure ammonium (+), (-)-tropicamide O-β-D-glucuronic acid, and said anticholinergic compound is (+,-)-tropicamide.

6. A process for separating ammonium (+)-tropicamide O-$\beta$-D-glucuronic acid and ammonium (-)-tropicamide O-$\beta$-D-glucuronic acid from ammonium (+),(-)-tropicamide O-$\beta$-D-glucuronic acid characterised by subjecting ammonium (+),(-)-tropicamide O-$\beta$-D-glucuronic acid to reversed phase liquid chromatography.

7. A process, according to claim 2, wherein the anticholinergic compound contains an ester moiety and which comprises:

(a) substantially removing esterases from liver microsomes;

(b) incubating said treated liver microsomes in the presence of:

(i) a suitable buffer to maintain the pH at about 7 to about 8.5;

(ii) the ester moiety-containing anticholinergic compound; and

(iii) uridine 5'-diphosphoglucuronic acid, for a sufficient time to conjugate the aglycon with glucuronic acid, and

(c) isolating the O-$\beta$-D-glucuronic acid of the ester moiety-containing anticholinergic compound.

8. A process, according to claim 2, wherein the anticholinergic compound contains an ester moiety and which comprises:

(a) substantially removing esterases from liver microsomes;

(b) incubating said treated liver microsomes in the presence of:

(i) a suitable buffer to maintain the pH at about 7 to about 8.5;

(ii) the ester moiety-containing anticholinergic compound;

(iii) uridine 5'-diphosphoglucuronic acid; and

(iv) a selective residual esterase inhibitor, and

(c) isolating the O-β-D- glucuronic acid of the ester moiety-containing anticholinergic compound.

9. A process, according to claim 1, for preparing the O-β-D- glucuronide of the compound having the primary alcohol moiety which comprises reacting a solution of D-glucuronic acid with a solution of said compound, and a solution of β-glucuronidase, for a time sufficient to form the O-β-D-glucuronide, and isolating said glucuronide.

10. A product which is (a) the O-β-D-glucuronic acid of a compound having a primary alcohol moiety, when prepared by a process according to claim 1 or claim 2; or (b) a base addition salt thereof.

11. The product of claim 10, wherein said O-β-D-glucuronic acid is any one of:

(i) (+),(-)-tropicamide O-β-D-glucuronic acid in its essentially pure form;

(ii) (+)-tropicamide O-β-D-glucuronic acid; or

(iii) (-)-tropicamide O-β-D-glucuronic acid.

12. A product which is (a) the O-β-D-glucuronic acid of an ester moiety-containing anticholinergic compound having a primary alcohol moiety, when prepared by a process according to claim 1 or claim 2; or

(b) a base addition salt thereof.

13. The product of claim 12, wherein said O-β-D-glucuronic acid is:

(i) scopolamine O-β-D-glucuronic acid;

(ii) hyoscyamine O-β-D-glucuronic acid; or

(iii) atropine O-β-D-glucuronic acid.

European Patent Office

# EUROPEAN SEARCH REPORT

0095362

Application number

EP 83 30 2936

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 69, no. 9, 26th August 1968, page 3167, no. 34028e, Columbus, Ohio, USA K.H. SCHMIDT: "Metabolism of tropane alkaloids. VIII. Chemical analysis of (-)-scopolamine metabolism in several mammals" & HOPPE-SEYLER'S Z. PHYSIOL. CHEM. 1968, 349(6), 741-752 * Abstract * | 1,9,13-15 | C 12 P 19/44<br>C 07 H 15/26<br>C 07 H 7/02 |
| A | CHEMICAL ABSTRACTS, vol. 71, no. 5, 4th August 1969, page 182, no. 20633c, Columbus, Ohio, USA G. WERNER: "Metabolic analysis and autoradiographic characterization of 14C-labeled and tritiated atropine and scopolamine and their metabolites in some mammals" & STRAHLENTHERAPIE, SONDERB. 1968, 67, 365-375 * Abstract * | 1,9,13-16 | |
| | | | TECHNICAL FIELDS SEARCHED (Int. Cl. 3) |
| A | CHEMICAL ABSTRACTS, vol. 75, no. 7, 16th August 1971, page 196, no. 47062k, Columbus, Ohio, USA G. WERNER: "Autoradiographic studies on the mode of action of atropine" & NUCL.-MED., SUPPL. 1968, (Pub. 1970), no. 8, 59-64 * Abstract * | 1,9,13,16 | C 12 P<br>C 07 H |

--- -/-

The present search report has been drawn up for all claims

| Place of search | Date of completion of the search | Examiner |
|---|---|---|
| THE HAGUE | 29-08-1983 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0095362
Application number

EP 83 30 2936



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 91, no. 3, 16th July 1979, page 287, no. 16222p, Columbus, Ohio, USA G. CARLSON et al.: "Glucuronidation of 3alpha-hydroxysteroids in vitro" & J. STEROID BIOCHEM. 1979, 10(2), 161-165 * Abstract * --- | 1 | |
| A | CHEMICAL ABSTRACTS, vol. 90, no. 15, 9th April 1979, page 11, no. 114786z, Columbus, Ohio, USA W. DIETERLE et al.: "Preparative reversed-phase chromatography of polar and nonpolar metabolites on columns packed with micronized XAD-2 resin" & J. CHROMATOGR. 1979, 168(1), 27-34 * Abstract * --- | 5 | TECHNICAL FIELDS SEARCHED (Int. Cl. 3) |
| A | CHEMICAL ABSTRACTS, vol. 87, no. 23, 5th December 1977, page 222, no. 179713f, Columbus, Ohio, USA Y. GRAEF et al.: "Effect of sodium sulfate on the hydrolysis of steroid glucuronides and nonsteroid glucuronides with the beta-glucuronidase preparations from bovine liver, Helix pomatia and E. coli" & RINSHO KAGAKU 1977, 5(2), 182-188 * Abstract * ----- | 1 | |

The present search report has been drawn up for all claims

| Place of search | Date of completion of the search | Examiner |
|---|---|---|
| THE HAGUE | 29-08-1983 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82